# EUROPÄISCHE PATENTSCHRIFT

(11) **EP 1 995 585 B1**
(45) Veröffentlichungstag und Bekanntmachung des Hinweises auf die Patenterteilung: **11.08.2010**
(21) Anmeldenummer: 08450083.4
(22) Anmeldetag: 20.05.2008
(51) Int. Cl.: G01N 11/12, G01N 33/38

(54) **Verfahren zur Bestimmung der Qualität von Frischbeton**
Process for determining the quality of fresh concrete
Procédé de détermination de la qualité du béton frais

(30) Priorität: 24.05.2007 AT 8292007
(43) Veröffentlichungstag der Anmeldung: 26.11.2008
(73) Patentinhaber: Kirchdorfer Fertigteilholding GmbH, 2752 Wöllersdorf (AT)
(72) Erfinder: Liviu-Valeriu, Roman, 2752 Woellersdorf (AT)
(74) Vertreter: Gibler, Ferdinand

(56) Entgegenhaltungen:
- BE-A- 428 139
- DE-A1- 10 008 664
- DE-A1-102004 015 707
- FR-A- 1 081 281
- FR-A- 1 279 449
- GB-A- 389 179
- GB-A- 191 122 042
- A. K. SACHAN; C. V. S. KAMESWARA RAO: "A cone penetration test for workability of fibre reinforced concrete" MATERIALS AND STRUCTURES, Bd. 21, Nr. 6, November 1988 (1988-11), Seiten 448-452, XP008100549

## Beschreibung

Die Erfindung betrifft ein Verfahren zur Bestimmung der Qualität von Frischbeton, insbesondere von selbstverdichtendem Frischbeton, gemäß dem Oberbegriff des Patentanspruches 1.

Als Frischbeton wird nicht abgebundener, nicht erhärteter, verarbeitbarer und fließfähiger Beton bezeichnet.

Die Qualität des Frischbetons, insbesondere des selbstverdichtenden Frischbetons, bestimmt im Wesentlichen, ob der Frischbeton zur Verwendung, insbesondere zur Verwendung Vor-Ort, geeignet ist. Frischbeton mit nicht hinreichender Qualität ist für die Verwendung Vor-Ort nicht geeignet, da dieser ein Sicherheitsrisiko birgt und die Verwendung hohe Nacharbeitungskosten zur Folge haben kann. Deshalb muss die Qualität des Frischbetons vor dem Erhärten zuverlässig geprüft und bestimmt werden.

Die Qualität des Frischbetons wird in diesem Zusammenhang durch drei Parameter wesentlich beeinflusst. Es sind dies die Frischbetonrohdichte, die Fließeigenschaften sowie die Entlüftungs- und Sedimentierungsstabilität. Für jeden dieser drei Parameter sind Prüfmittel, Prüfmethoden und Kennwerte bekannt.

Die Veröffentlichung A cone penetration test for workability of fibre reinforced concrete zeigt eine Methode zur Bestimmung der Frischbetondichte.

Auch die DE 100 08 664 A1 beschreibt ein Verfahren zur Bestimmung der Dichte von Beton. Die FR 1 279 449 A behandelt die Messung der Dichte einer Gipsmasse mittels eines Eintauchkörpers.

Die DE 10 2004 015 707 A1 beschreibt ein Rheometer.

Die Frischbetonrohdichte wird beispielsweise mittels eines Behälters mit einem vorbestimmten Volumen gemessen, welches mit Frischbeton befüllt wird. Die Masse des Frischbetons in dem Behälter geteilt durch das vorbestimmte Volumen des Behälters ergibt die Frischbetonrohdicbte. So wird die Frischbetonrohdichte mittels eines Verfahrens zur Dichtebestimmung ermittelt.

Die Fließeigenschaften werden beispielsweise mittels eines Viskosimeters und/oder mittels des Versuchs in einer U-Box oder in einer L-Box ermittelt. In der U-Box oder der L-Box läuft der Beton nach dem Einfüllen durch ein Hindernis von einer Kammer in eine zweite Kammer. Dabei kann das Fließen beobachtet, die Fließzeit gemessen und die sich in einer Gleichgewichtslage einstellende Höhendifferenz und/oder Winkelabweichung von der Waagerechten der Betonoberfläche ermittelt werden. Für die Ermittelung des so genannten Blockierverhaltens des selbstverdichtenden Betons ist ebenso die Prüfung mittels eines Blockierrings bekannt. Die Fließeigenschaften werden mittels einem Fließkanalversuch umfassend eine U-Box oder eine L-Box ermittelt. Ebenso ist ein Versuch mit dem Beton-Viskosimeter bekannt.

Die Entlüftungs- und Sedimentienmgsstabilität wird üblicherweise über Visualisierungsmethoden ermittelt. Dazu wird Beton in einem Behälter gefüllt und nach dem Erhärten in Längsrichtung zersägt. Die Sedimentierung des groben Korns kann visuell festgestellt werden. Ab einer gewissen Neigung zur Sedimentierung, also einer geringen Sedimentierungsstabilität, ist der Frischbeton nicht zur Verwendung als selbstverdichtender Frischbeton einzustufen.

Nachteilig daran ist, dass diese Prüfmethoden aufwendig sind. Vor allem die Versuche zur Bestimmung der Fließeigenschaften und der Entlüftungs- und Sedimentierungsstabilität werden deshalb meist im Labor und lediglich bei der Entwicklung neuer Zusammensetzungen des Betons angewandt. Oftmals sind die erforderlichen Messgeräte empfindlich und können nicht im Freien oder im Baustellenbereich verwendet werden. Ebenso ist der Bedienungsaufwand derartiger Messgeräte hoch, sodass diese lediglich von geschultem Personal betrieben werden können. Vor-Ort und/oder im Baustellenbereich finden diese Prüfmittel daher kaum Verwendung.

Nachteilig daran ist, dass vor allem die Bestimmung der Entlüftungs- und Sedimentierungsstabilität erst nach dem abbinden des Frischbetons erfolgen kann, sodass ein - mittlerweile erhärtetes - Bauteil umfassen die entsprechende Betoncharge abgerissen werden müsste.

Ebenso ist daran nachteilig, dass mehrere Einzelprüfungen zur Feststellung der ausreichenden Qualität des Frischbetons notwendig sind, wodurch sich der Aufwand zur Feststellung der Qualität des Frischbetons weiter erhöht.

Aufgabe der Erfindung ist es daher ein Verfahren zur Bestimmung der Qualität von Frischbeton anzugeben, mit welchem die genannten Nachteile vermieden werden können, mit welchem Vor-Ort an der Baustelle eine qualifizierte Aussage über die Qualität des Frischbetons getroffen werden kann und welches in einer Baustellenumgebung mit großer Zuverlässigkeit eingesetzt werden kann.

Erfindungsgemäß wird dies durch die Merkmale des Patentanspruches 1 erreicht.

Durch das Eintauchen kann sowohl auf die Fließeigenschaften als auch auf die Frischbetonrohdichte rückgeschlossen werden. Das Eintauchen kann direkt Vor-Ort und insbesondere an einem bereits befüllten Betonbauteil und/oder in einer Schalung erfolgen. Dadurch kann die Qualität des Frischbetons direkt am Bauteil und bei der Verarbeitung bestimmt werden, wodurch direkt auf die Qualität des Bauteils rückgeschlossen werden kann. Dies ermöglicht die unmittelbare Qualitätskontrolle des Frischbetons und des Bauteiles.

Das Eintauchen kann auch unmittelbar vor der Befüllung des Bauteiles und/oder der Schalung erfolgen, sodass die Verarbeitung einer möglicherweise unzureichenden Qualität des Frischbetons verhindert werden kann. Derart können Folgekosten durch den Austausch des Frischebctons und/oder des Bauteiles vermieden werden. Da die Prüfung der Qualität des Frischebetons unmittelbar vor der Verarbeitung erfolgen kann, ist weiterhin die Möglichkeit auf den Rückschluss der Qualität des Bauteiles gegeben.

Weitere Aufgabe ist es ein Prüfmittel zur Bestimmung der Qualität von Frischbeton der eingangs genannten Art anzugeben, mit welchem die genannten Nachteile vermieden werden können, mit welchem Vor-Ort an der Baustelle eine qualifizierte Aussage über die Qualität des Frischbetons getroffen werden kann, welches einfach und kostenschonend in der Herstellung und im Einsatz ist und welches in einer Baustellenumgebung mit großer Zuverlässigkeit eingesetzt werden kann.

Die Unteransprüche, welche ebenso wie der Patentanspruch 1 gleichzeitig einen Teil der Beschreibung bilden, betreffen weitere vorteilhafte Ausgestaltungen der Erfindung. Vorteilhafterweise kann in Weiterbildung der Erfindung vorgesehen sein, dass das Prüfmittel einen Schwimmkörper und/oder einen Stabilisierungskörper umfasst.

Das derart ausgebildete Prüfmittel kann mit ausreichender Stabilität für den Einsatz im Außenbereich, im Baustellenbereich und dem Kontakt mit dem Frischbeton mit geeigneter mechanischer und chemischer Stabilität ausgebildet sein. Derart wird die zuverlässige Verwendung des Prüfmittels gewährleistet.

Dieses Prüfmittel kann ebenso einfach und mit geringem Schulungsaufwand zuverlässig verwendet werden, weshalb es besonders für den Gebrauch für Vor-Ort und/oder im Baustellenbereich geeignet ist.

Die Feststellung der hinreichenden Qualität des Frischbetons, insbesondere des selbstverdichtenden Frischbetons, kann in diesem Zusammenhang auch mit einem einzigen Prüfmittel erfolgen, wodurch ein geringer Prüfaufwand gewährleistet ist.

Die Erfindung wird unter Bezugnahme auf die beigeschlossene Zeichnung, in welcher lediglich eine bevorzugte Ausführungsform beispielhaft dargestellt ist, näher beschrieben.

Die Fig. zeigt in Gebrauchslage und in einer seitlichen Ansicht ein Prüfmittel 1 zur Bestimmung der Qualität von Frischbeton 9, insbesondere von selbstverdichtendem Frischbeton 9, wobei zur einfachen Bestimmung der Qualität von Frischbeton 9, insbesondere Vor-Ort, im Baustellenbereich und/oder im Bauteil umfassend den Frischbeton 9, sowie zur Bestimmung mittels eines einzigen Prüfmittels 1 vorgesehen ist, dass das Prüfmittel 1 einen Eintauchkörper 2 zum Eintauchen in den Frischbeton 9 und zur Ausbildung einer Gleichgewichtslage im Frischbeton 9 umfasst.

In diesem Zusammenhang ist die Gleichgewichtslage eine relativ zum Frischbeton 9 ortsfeste und relativ zum Frischbeton 9 impulsfreie Lage, insbesondere Ruhelage, des Prüfmittels 1. In der Gleichgewichtslage im Frischbeton 9 heben die, auf das Prüfmittel 1 wirkenden, Auftriebskräfte und die Gewichtskraft des Prüfmittels 1 einander auf.

Zur Prüfung und/oder zur Feststellung der Qualität des Frischbetons 9, insbesondere des selbstverdichtenden Frischbetons 9, wird das Prüfmittel 1 mit dem - in Gebrauchslage gesehen - unteren Ende an die Oberfläche des zu prüfenden Frischbetons 9 gehalten. Der zu prüfende Frischbeton 9 befindet sich dabei in einem Behältnis. Das Behältnis kann beispielsweise als eine Mischtrommel, ein Becken, eine Grube oder aus Schalungswänden ausgebildet sein.

Sämtliche örtliche Angaben, wie oben, unten, horizontale Erstreckung oder dergleichen, beziehen sich - sofern nicht anders angegeben - im Weiteren auf die in der Fig. dargestellte Gebrauchslage des Prüfmittels 1.

Vorteilhafterweise erfolgt die Prüfung in hinreichendem Abstand zu sämtlichen seitlichen Wänden des, mit Frischbeton 9, befüllten Behältnis. Vorzugsweise befindet sich an der zu messenden Stelle Frischbeton 9 mit hinreichender vertikaler Erstreckung im Behältnis. Dadurch ist gewährleistet, dass bei der Feststellung der Qualität des Frischbetons 9 keine Seitenwand- und/oder Bodeneinflüsse mitbestimmt werden.

Der hinreichende Mindestabstand des Prüfmittels 1 zu den Seitenwänden beträgt bei der Prüfung vorzugsweise in etwa dem doppelten der horizontalen Erstreckung des Prüfmittels 1. Bei einem Prüfmittel 1 mit einem maximalen horizontalen Durchmesser von beispielsweise 0,1m, sollte vorteilhafterweise ein Mindestabstand zur seitlichen Berandung des Frischbetons 9, insbesondere den seitlichen Wänden des Behältnis, von 0,2m nicht unterschritten werden.

Die minimale vertikale Erstreckung des Frischbetons 9 an der zu prüfenden Stelle beträgt vorteilhafterweise das 1,25fache der vertikalen Erstreckung des Eintauchkörpers 2. Bei einem Prüfmittel 1 umfassend den Eintauchkörper 2 mit einer vertikalen Erstreckung von beispielsweise 0,45m, sollte an der Stelle oder an der Position der Prüfung vorteilhafterweise die horizontale Erstreckung des Frischbetons 9 von der Frischbetonoberfläche 91 zu einer der Frischbetonoberfläche 91 gegenüberliegenden Berandung des Frischbetons 9, insbesondere des Bodens des Behältnis, in etwa 0,56m nicht unterschreiten.

Das Prüfmittel 1 kann vorteilhafterweise mit einer Gesamtmasse zwischen 0,5kg und 5kg, insbesondere zwischen 1kg und 1,5kg ausgebildet sein. Das Prüfinittel 1 kann mit einer - in Gebrauchslage insbesondere horizontalen - Längserstreckung zwischen 0,2m und 0,8m, insbesondere zwischen 0,35m und 0,55m ausgebildet sein. Dadurch kann das Prüfmittel 1 durch eine Person einfach und präzise bedient werden und der Transport, die Lagerung und/oder die Reinigung des Prüfmittels 1 kann einfach und kostenschonend erfolgen.

Das Prüfmittel 1 kann vorteilhafterweise Metalle und/oder Metalllegierungen umfassen, insbesondere im Wesentlichen aus Metallen und/oder Metalllegierungen ausgebildet sein. Besonders vorteilhaft sind Stahl, Bronze, Blei und/oder Legierungen umfassend diese Metalle. Diese Materialien weisen eine hohe mechanische und chemische Beständigkeit auf. Das Prüfinittel 1 kann einfach und kostenschonend hergestellt werden.

Das dargestellte Prüfmittel 1 umfasst einen Eintauchkörper 2. Der Eintauchkörper 2 ist zum Eintauchen in den Frischbeton 9 vorbestimmt, wobei die komplette Oberfläche des Eintauchkörpers 2 geschlossen sein kann. Alternativ kann der Eintauchkörper 2 am oberen Ende offen ausgestaltet sein.

Der Eintauchkörper 2 umfasst vorteilhafterweise einen Schwimmkörper 3. Dadurch ist das Prüfmittel 1 zum Schwimmen und zur Ausbildung der Gleichgewichtslage im Frischbeton 9 ausgebildet. Vorteilhafterweise kann der Schwimmkörper 3 als Hohlkörper ausgebildet sein. In diesem Fall kann der Schwimmer 3 auch aus Materialien mit größerer Dichte als Frischbeton 9, insbesondere umfassend Metalle und/oder Metalllegierungen ausgebildet sein. Dadurch kann die mechanische und chemische Stabilität des Schwimmkörpers 3 gewährleistet sein. Im inneren des Schwimmkörpers 3 kann ein geschäumter Werkstoff, insbesondere Kunststoff, vorgesehen sein.

Vorteilhafterweise kann vorgesehen sein, dass der Schwimmkörper 3 - in Gebrauchslage gesehen - mit einer nach unten gerichteten Spitze 31 ausgebildet ist. Dadurch ist ein geringer Eintauchwiderstand des Schwimmkörpers 3 gegeben, da die Spitze 31 nur einen geringen Querschnitt aufweist. Die Dauer bis zur Ausbildung der Gleichgewichtslage wird nur wenig durch die Spitze beeinflusst.

Vorteilhafterweise kann vorgesehen sein, dass der Schwimmkörper 3 - in Gebrauchslage gesehen - einen von unten nach oben stetig ansteigenden horizontalen Querschnitt aufweist, insbesondere kegel- oder pyramidenförmig ausgebildet ist. Dadurch kann der Messbereich der Rohdichte des Frischbetons 9 vergrößert werden.

Mit Oberflächengeometrie und/oder der Oberflächenstruktur des Schwimmkörpers 3 kann weiters auch die Eintauchgeschwindigkeit und die Dauer bis zur Ausbildung der Gleichgewichtslage vorgebbar angepasst werden. Vorteilhafterweise ist die Oberfläche derart ausgebildet, dass es beim Eintauchen des Prüfmittels 1 zu keinem bzw. nur geringem Schwingen um die Gleichgewichtslage kommt, wobei ebenso eine langsame Annäherung an die Gleichgewichtslage vermieden werden kann. Ein von unten nach oben stetig ansteigender horizontaler Querschnitt erfüllt diese Kriterien, womit die schnelle und zuverlässige Prüfung der Qualität des Frischbetons 9 gewährleistet ist.

Bei vorbestimmter Konsistenz des zu prüfenden Frischbetons 9, kann der Schwimmkörper 3 vorteilhafterweise auch mit konstantem horizontalem Querschnitt, halbkugelförmig, kugelförmig oder tropfenförmig ausgebildet sein.

Der Eintauchkörper 2 umfasst vorteilhafterweise auch einen Stabilisierungskörper 4. Der Stabilisierungskörper 4 ist vorteilhafterweise in der Gebrauchslage unterhalb des Schwimmkörpers 3, insbesondere am unteren Ende des Prüfmittels 1, angeordnet. Die Lage des Prüfmittels 1 in dem Frischbeton 9 wird dadurch stabilisiert und eine hohe Zuverlässigkeit der Prüfung wird gewährleistet.

Der Stabilisierungskörper 4 ist vorteilhafterweise in diesem Zusammenhang als Sinkkörper ausgebildet. Dadurch wird das Umkippen verhindert und die vertikale Ausrichtung des Prüfmittels sichergestellt.

Zur Ausbildung von beim Eintauchen konstanten Strömungsverhältnissen über den horizontalen Umfang des Prüfmittels 1, kann vorteilhafterweise vorgesehen sein, dass der Stabilisierungskörper 4 - in Gebrauchslage gesehen - um eine Vertikalachse 11 radialsymmetrisch, bevorzugt rotationssymmetrisch, vorzugsweise tropfenförmig und/oder kugelförmig ausgebildet ist. Die radialsymmetrische und/oder rotationssymmetrische Geometrie des Stabilisierungskörpers 4 ist vorteilhaft, da derart die Ausbildung eines Drehmomentes im Prüfmittel 1 beim Eintauchen des Prüfmittel 1 in den Frischbeton 9 verhindert werden kann. Die tropfenförmige und/oder die kugelförmige Geometrie des Stabilisierungskörpers 4 ist vorteilhaft, da derart bei geringer Oberfläche und geringem Eintauchwiderstand des Stabilisierungskörpers 4 ein großes Volumen und eine große Masse ausgebildet werden kann. Derart kann ein Großteil der Masse des Prüfmittels 1 im Stabilisierungskörper 4 ausgebildet sein, sodass die Stabilisierung des Prüfmittels 1 beim Eintauchen besonders gewährleistet ist. Dies gewährleistet die sichere Stabilisierung des Prüfmittels 1 während des Eintauchens und während der Feststellung der Qualität des Frischbetons 9.

In vorteilhafter Weiterbildung kann vorgesehen sein, dass der Stabilisierungskörper 4 als Vollkörper, umfassend ein Metall oder eine Metalllegierung, insbesondere Stahl, Bronze und/oder Blei, ausgebildet ist. Dadurch kann der Massenschwerpunkt im unteren Bereich des Prüfmittels 1 ausgebildet und die Stabilisierwirkung hoch sein. Der Stabilisierungskörper 4 kann mit geringen Außenabmessungen ausgebildet sein, sodass dieser einen nur geringen Widerstand beim Eintauchen bewirkt. Dies ermöglicht hohe Eintauchgeschwindigkeiten und kurze Messzeiten.

Der Stabilisierungskörper 4 kann an der nach unten gerichteten Fläche alternativ auch unterschiedlich zu der nach oben gerichteten Fläche ausgebildet sein. Beispielsweise ist die bei einer halbkugelförmigen Ausgestaltung des Stabilisierungskörpers 4 der Fall. Bei dieser Ausgestaltung ergeben sich bei der Bewegung des Prüfmittels 1 im Frischbeton 9 nach unten und bei der Bewegung des Prüfmittels 1 im Frischbeton nach oben unterschiedliche Strömungswiderstände. Dies kann die schnelle Ausbildung der Gleichgewichtslage begünstigen und Einschwingvorgänge gezielt einseitig dämpfen.

Dadurch, dass dar Stabilisierungsköper 4 Stahl, Bronze und/oder Blei umfasst, insbesondere im Wesentlichen aus Stahl, Bronze und/oder Blei ausgebildet ist, kann die hohe mechanische und chemische Beständigkeit gewährleistet sein.

Vorteilhafterweise können am Schwimmkörper 3 an der Außenfläche Messmarken 32 angeordnet sein, insbesondere eine vorgebbare Mehrzahl von Messmarken 32 angeordnet sein. Diese Messmarken 32 ermöglichen ein einfaches Ablesen der Eintauchtiefe des Prüfmittels 1 im Frischbeton 9. Die Messmarken 32 können dazu in vorbestimmten Abständen angeordnet sein. Insbesondere können die Messmarken 32 zueinander äquidistant angeordnet sein, wobei sowohl eine quantitative als auch eine qualitative Aussage über die Rohdichte des Frischbetons 9 getroffen werden kann. In vielen Fällen kann die qualitative Prüfung der Rohdichte ausreichen, um eine hinreichende Aussage über die Qualität des Frischbetons 9 zu erhalten. Dazu können zwei Messmarken an der Oberfläche des Schwimmkörpers 3 ausgebildet sein, wobei die hinreichende Qualität - in Hinblick auf die Rohdichte - gegeben ist, wenn die Frischbetonoberfläche 91 in der Gleichgewichtslage des Prüfmittels 1 zwischen diesen beiden Messmarken angeordnet ist.

Alternativ können die Messmarken 32 zur Darstellung äquidistanter Messwerte ausgebildet sein. Dabei können die Messmarken 32 vorteilhafterweise auf das Volumen, das Gewicht und die Prüfmitteloberfläche abgestimmt sein.

Die Messmarken 32 können als Einkerbungen, als Aufdrucke, als Linien oder als Punkte und/oder umfassend eine oder mehrere Farben ausgebildet sein, um auch bei schlechten Lichtverhältnissen eine gute Ablesbarkeit zu gewährleisten. Insbesondere können die Messmarken 32 Erhebung umfassen, womit die Ablesbarkeit auch bei betonbedeckter Oberfläche des Eintauchkörpers 2 gewährleistet ist.

Vorteilhafterweise ist am oberen Ende des Prüfmittels 1 ein Haltemittel 5 angeordnet. Das in der Fig. dargestellte Haltemittel 5 kann stabförmig ausgebildet sein. Die Längserstreckung des Haltemittels 5 ist - in der Gebrauchslage - insbesondere im Wesentlichen parallel zu einer Vertikalachse 11. Insbesondere kann sich das Haltemittel 5 vom oberen Ende des Prüfmittels 1 bis zum oberen Ende des Eintauchkörpers 2 erstrecken.

Das Haltemittel 5 kann sich vorteilhafterweise vom oberen Ende des Prüfmittels 1 zum Stabilisierungskörper 4 erstrecken. Das Haltemittel 5 kann den Stabilisierungskörper 4 und/oder der Schwimmkörper 3 in der Lage fixieren, wobei das Haltemittel 5 als Versteifungselement des Prüfmittels 1 ausgebildet sein kann. Insbesondere kann das Haltemittel 5 um eine Symmetrieachse 12 des Prüfmittels 1 ausgebildet sein. Dadurch kann sowohl der Schwimmkörper 3 als auch der Stabilisierungskörper 4 in der Lage im Prüfmittel 1 fixiert und/oder stabilisiert werden.

Mit dem Haltemittel 5 wird das Prüfmittel 1 vor der Feststellung der Qualität des Frischbetons 9 in die Gebrauchslage gebracht. Dazu wird das Prüfinittel 1 am oberen Ende, insbesondere am Haltemittel 5, mittels einer Haltevorrichtung und/oder durch eine Person, gehalten.

Vorteilhafterweise kann vorgesehen sein, dass das Prüfmittel 1 eine Trimmgewichtsaufnahme 6 zur Anordnung eines Trimmgewichts 62 umfasst. Das vorteilhafterweise an der Trimmgewichtsaufnahme 6 angebrachte Trimmgewicht 62 kann die Gewichtsymmetrie um die Symmetrieachse 12 beeinflussen. Mittels dem Trimmgewicht 62 können beim Gebrauch möglicherweise störende Abweichungen der Lage des Massenschwerpunktes des Prüfmittels 1 in der Höhe und von der Symmetrieachse 12 beeinflusst werden. Dadurch kann die vorbestimmte Lage des Massenschwerpunktes des Prüfmittels 1 über die gesamte Lebensdauer des Prüfmittels 1 gewährleistet sein und/oder eine Temperaturkompensation durchgeführt werden.

Vorteilhafterweise kann die Trimmgewichtsaufnahme 6 - in Gebrauchslage gesehen - am oberen oder unteren Ende des Schwimmkörpers 3, insbesondere am oberen Ende des Schwimmkörpers 3, angeordnet sein. Dadurch ist die Trimmgewichtsaufnahme 6 von außen gut zugänglich, sodass das Einstellen des Trimmgewichtes 62 oder der Trimmgewichte 62 besonders einfach erfolgen kann. Besonders wenn zwei Trimmgewichtsaufnahmen 6, insbesondere eine in der oberen Hälfte des Prüfmittels 1 und eine in der unteren Hälfte des Prüfmittels 1, vorhanden sind, kann eine Positionierung des Masseschwerpunkts des Prüfmittels 1 besonders einfach und präzise erfolgen.

Die Einstellung des Gesamtgewichts und/oder des Masseschwerpunktes mittels der Trimmgewichte 62 kann bei dieser Ausgestaltung einfach und Vor-Ort und/oder im Baustellenbereich erfolgen. Zusätzlich oder alternativ kann innerhalb des Schwimmkörpers 3 eine Trimmgewichtaufnahme 6 angeordnet sein. Diese Trimmgewichtaufnahme 6 kann von außen nicht oder lediglich durch ein Zerlegen des Prüfmittels 1 zugänglich sein.

In besonders vorteilhafter Weiterbildung kann vorgesehen sein, dass das Prüfmittel 1 um eine Vertikalachse 11 radialsymmetrisch, insbesondere rotationssymmetrisch, ausgebildet ist, und dass die Vertikalachse 11 eine Symmetrieachse 12 des Prüfmittels 1 ist. Dadurch kann der Massenschwerpunkt ebenfalls in der Symmetrieachse 12 liegen. Dadurch können die sich beim Eintauchen des Eintauchkörpers 2 des Prüfmittels 1 einstellenden Strömungsverhältnisse rotationssymmetrisch ausbilden. Dadurch kann das Prüfmittel 1 in der Gleichgewichtslage im Wesentlichen mit einer vertikalen Längserstreckung ausgebildet sein. Dadurch kann während des Eintauchens und bei der Gleichgewichtslage des Prüfmittels 1 im Frischbeton 9 die Symmetrieachse 12 des Prüfmittels 1 im Wesentlichen vertikal ausgebildet sein. Dadurch kann ein Kippen des Prüfmittels 1 beim Eintauchen in den Frischbeton 9 vermieden werden. Derart können die Messung störende, kaum quantifizierbare Einflüsse und/oder systematische Messfehler vermieden werden. Die Messung, Prüfung und/oder Feststellung der Qualität des Frischbetons 9 kann mit hoher Reproduzierbarkeit und hoher Zuverlässigkeit durchgeführt werden.

Die Erfindung betrifft ein Verfahren zur Bestimmung der Qualität von Frischbeton 9, insbesondere von selbstverdichtendem Frischbeton 9, wobei zur einfachen Bestimmung Qualität von Frischbeton 9 Vor-Ort, im Baustellenbereich und/oder im Bauteil umfassend den Frischbeton 9 vorgeschlagen wird, dass ein Prüfmittel 1 mit einem Eintauchkörper 2 zum Eintauchen in den Frischbeton 9, insbesondere ein Prüfinittel nach einem der Ansprüche 1 bis 14, mit der - in Gebrauchslage gesehen - Prüfmittelunterseite 13 an eine Frischbetonoberfläche 9 angehalten wird, dass das Prüfmittel 1 freigemacht wird zum Eintauchen in den Frischbeton 9, dass eine Eintauchtiefe des Prüfmittels 1 im Wesentlichen nach Ausbildung einer Gleichgewichtslage gemessen wird, und dass aus der Eintauchtiefe ein Wert für die Rohdichte des Frischbetons 9 ermittelt wird.

Mit diesem Verfahren kann mit einfachen Mitteln und mit wenigen Verfahrensschritten auf die Qualität des Frischbetons 9 rückgeschlossen werden. Insbesondere kann innerhalb eines kurzen Zeitintervalls geprüft werden ob der Frischbeton 9, insbesondere der selbstverdichtende Frischbeton 9, zur Verarbeitung und zur Verwendung geeignet ist.

Das Verfahren kann bevorzugt mit einem Prüfmittel 1 gemäß der in der Fig. dargestellten bevorzugten Ausführungsform durchgeführt werden.

Das Prüfmittel 1 wird vor Beginn des erfindungsgemäßen Verfahrens im Bereich des oberen Endes ortsfest und momentfrei, also rotierbar, gehalten. Das Prüfmittel 1 wird sich dabei in die Gebrauchslage drehen, sodass die Prüfmittelunterseite 13 in Richtung Frischbeton 9 und in Richtung Frischbetonoberfläche 91 gerichtet ist. Dadurch kann der Schwerpunkt des Prüfmittels 1 mit der Symmetrieachse 12 zusammenfallen, sodass im Wesentlichen beim Eintauchen im Frischbeton 9 keine die Messung und/oder Prüfung beeinflussenden Momente im Prüfmittel 1 wirken.

Dabei kann vorteilhafterweise vorgesehen sein, dass am oberen Ende des Haltemittels 5 eine Halteschnur vorgesehen ist, wodurch das Prüfmittel 1 vorteilhafterweise momentfrei in der Gebrauchslage gehalten werden kann.

Das Prüfmittel 1 wird mit dessen Prüfmittelunterseite 13 vorteilhafterweise bei Beginn der Prüfung an der Frischbetonoberfläche 91 angehalten. Dadurch ist das Prüfmittel beim Beginn der Prüfung ortsfest und ohne Relativimpuls zum Frischbeton 9 angeordnet. Die im Wesentlichen vorbestimmbare, insbesondere vorbestimmte, Gewichtskraft des Prüfmittels 1 als auch die sich mit dem Eintauchen in den Frischbeton 9 ausbildende Auftriebskraft, bestimmen die Ausbildung der Gleichgewichtslage.

Nach Einstellen der Gleichgewichtslage kann der Wert für die Rohdichte des Frischbetons 9 ermittelt werden. Dies kann sowohl qualitativ als auch quantitativ erfolgen. Die qualitative Bestimmung des Wertes ist in diesem Zusammenhang die Einteilung in Kategorien, insbesondere in die Kategorien Gutqualität der Rohdichte und Schlechtqualität der Rohdichte. Die quantitative Bestimmung des Wertes ist in diesem Zusammenhang die Ermittlung einer Messgröße und insbesondere die Zuordnung eines Messwertes einer vorbestimmten Einheit, insbesondere der Einheit der Dichte.

In vorteilhafter Weiterbildung der Erfindung kann vorgesehen sein, dass der Wert für die Rohdichte des Frischbetons 9 direkt an einer Messmarke des Schwimmkörpers 3 abgelesen wird. Dies ermöglicht eine einfache quantitative oder qualitative Bestimmung der Bestimmungsgröße. Es können vorteilhafterweise mehrere Messmarken 32 ausbildet sein, wobei eine hohe Ablesegenauigkeit gewährleistet werden kann.

In besonders vorteilhafter Weiterbildung der Erfindung kann vorgesehen sein, dass die Zeit bis zum Ausbilden der Gleichgewichtslage des Prüfmittels 1 im Frischbeton 9 gemessen wird, und dass aus der gemessenen Zeit ein Wert für die Fließeigenschaften, insbesondere die Viskosität, des Frischbetons 9 ermittelt wird. Dadurch kann auf die Viskosität und die Fließeigenschaften des geprüften Frischbetons 9, insbesondere des selbstverdichtenden Frischbetons 9, rückgeschlossen werden. Der vorbestimmbare, insbesondere vorbestimmte, Eintauchwiderstand des Prüfmittels 1, insbesondere des Eintauchkörpers 2 bestimmen das Zeitintervall bis zur Ausbildung der Gleichgewichtslage des Prüfmittels 1 im Frischbeton 9.

Bei gleichem Prüfmittel 9 jedoch größerem Zeitintervall, welches beispielsweise in Sekunden gemessen wird, kann derart auf einen höheren Eintauchwiderstand und/oder auf eine höhere Viskosität des Frischebetons 9 rückgeschlossen werden. Das Zeitintervall, also die Eintauchdauer, ist dabei von der Viskosität des Frischbetons und der Prüfmittelgeometrie abhängig.

Vorteilhafterweise kann zur Temperaturkompensation eine Temperatur des Frischbetons 9 gemessen werden. Da die Viskosität des Frischbetons 9 mit der Temperatur des Frischbetons 9 zusammenhängen kann, so kann mittels Korrekturwerten, insbesondere Korrekturtabellen die Temperaturkompensation durchgeführt werden. Die Messgenauigkeit und/oder die Prüfgenauigkeit kann derart auch bei unterschiedlichen Jahreszeiten und bei unterschiedlichen Außentemperaturen und Frischbetontemperaturen vergleichbar und/oder mit hoher Zuverlässigkeit durchgeführt werden.

Gemäß der Erfindung ist vorgesehen, dass das erfindungsgemäße Verfahren nach einer vorgebbaren Zeitdauer, bevorzugt nach 5 bis 10 Minuten, insbesondere nach 7 bis 8 Minuten, zumindest ein zweites Mal durchgeführt wird. Dabei kann insbesondere vorgesehen sein, dass das Verfahren an gleicher Position im Frischbeton 9 durchgeführt wird. Dadurch kann mit einfachen Methoden und mit einem zuverlässigen Prüfverfahren eine Entlüftungs- und/oder eine Sedimentierungsstabilität geprüft werden.

Insbesondere kann bei den zumindest zwei - im Wesentlichen - an gleicher Position im Frischbeton 9 und zu unterschiedlichen Zeitpunkten durchgeführten Prüfungen und/oder Bestimmungen der Qualität des Frischbetons, die Rohdichte des Frischbetons 9 und die Zeitdauer von Loslassen des Prüfmittels 1 bis zur Ausbildung der Gleichgewichtslage des Prüfmittels 1 ermittelt werden. Derart werden vier Messwerte ermittelt. Dadurch können mit besonderer Genauigkeit Rückschlüsse auf die Rohdichte, das Fließverhalten sowie die Entlüftungs- und/oder eine Sedimentierungsstabilität des Frischbetons 9 gewonnen werden.

Bevorzugterweise kann in einem zusätzlichen Verfahrensschritt vor oder nach der Bestimmung der Qualität von Frischbeton 9 eine Trichterauslaufzeit-Prüfmethode angewandt werden. Die Trichterauslaufzeit-Prüfmethode, mittels welcher ebenfalls auf bestimmte Fließeigenschaften des Frischbetons 9 rückgeschlossen werden kann, ermöglicht die Bestimmung eines zusätzlichen Kennwertes des Frischbetons 9. Durch die Korrelation des bei der Bestimmung der Eintauchzeit des Prüfmittels 1 gewonnen Kennwertes und/oder Messwertes mit dem aus der Trichterauslaufzeit-Prüfmethode können zusätzliche Rückschlüsse auf die Fließeigenschaften des Frischbetons 9 gewonnen werden. Die hohe Zuverlässigkeit und die hohe Messgenauigkeit kann über einen weiten Bereich unterschiedlicher Betonsorten, beispielsweise Leichtbeton, Normalbeton und/oder Schwerbeton, sowie Ortbeton und/oder Transportbeton im nicht erhärteten nicht abgebundenen Zustand gewährleistet werden.

Insbesondere wenn das erfindungsgemäße Verfahren zumindest zweimal durchgeführt wird, kann die Trichterauslaufzeit-Prüfmethode ebenso zumindest zweimal durchgeführt werden. Die nunmehr ermittelten sechs Kennwerte können die Prüfgenauigkeit und die Zuverlässigkeit der Bestimmung der Qualität des Frischbetons 9 erhöhen.

Zur Bestimmung von Abweichungen innerhalb des Bauteiles kann vorgesehen sein, dass das erfindungsgemäße Verfahren an mehreren Stellen im - das Bauteil ausbildenden - Frischbeton 9 durchgeführt wird.

Durch die Durchführung von im Wesentlichen zeitgleichen und/oder sofort hintereinander durchgeführten, mehreren Bestimmungen und die Bildung eines Mittelwertes der ermittelten qualitativen und/oder quantitativen Werte und/oder Kennwerte dieser Bestimmungen, kann die hohe Genauigkeit der Ermittelung und die hohe Zuverlässigkeit, vor allem gegenüber statistischen Schwankungen der Mess- und/oder Prüfungsgenauigkeit, gewährleistet werden.

Das Prüfinittel 1 und das erfindungsgemäße Verfahren können insbesondere durch unterschiedliche Personen und/oder Personenkreise mit hoher Zuverlässigkeit und hoher Genauigkeit bei geringem zeitlichem und personellem Aufwand durchgeführt werden.

Mit automatisierten Systemen kann die Prüfung automatisiert und/oder teilautomatisiert durchgeführt werden. Die Werte, Messwert und/oder die Kenngrößen können dabei mittels elektronischer Datenerfassung erfasst werden.

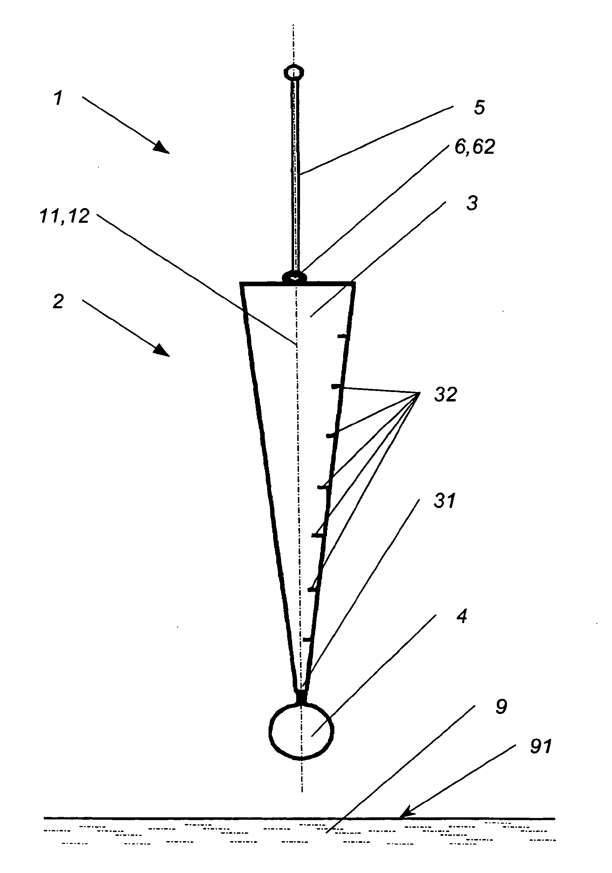

## Patentansprüche

1. Verfahren zur Bestimmung der Qualität von Frischbeton (9), insbesondere von selbstverdichtendem Frischbeton (9), wobei ein Prüfmittel (1) mit einen Eintauchkörper (2) zum Eintauchen in den Frischbeton (9) mit der - in Gebrauchslage gesehen - Prüfmittelunterseite an eine Frischbetonoberfläche (91) angehalten wird, wobei das Prüfmittel (1) freigemacht wird zum Eintauchen in den Frischbeton (9), wobei eine Eintauchtiefe des Prüfmittels (1) im Wesentlichen nach Ausbildung einer Gleichgewichtslage gemessen wird, und wobei aus der Eintauchtiefe ein Wert für die Rohdichte des Frischbetons (9) ermittelt wird, **dadurch gekennzeichnet, dass** zur Prüfung der Entlüftungs- und/oder Sedimentierungsstabilität das Verfahren nach einer vorgebbaren Zeitdauer zumindest ein zweites Mal durchgeführt wird.

2. Verfahren nach Anspruch 1, **dadurch gekennzeichnet, dass** das Verfahren nach 5 bis 10 Minuten zumindest ein zweites Mal durchgeführt wird.

3. Verfahren nach Anspruch 2, **dadurch gekennzeichnet, dass** das Verfahren nach 7 bis 8 Minuten zumindest ein zweites Mal durchgeführt wird.

4. Verfahren nach einem der Ansprüche 1 bis 3, **dadurch gekennzeichnet, dass** das Verfahren an gleicher Position im Frischbeton (9) ein zweites Mal durchgeführt wird.

5. Verfahren nach einem der Ansprüche 1 bis 4, **dadurch gekennzeichnet, dass** die Zeit bis zum Ausbilden der Gleichgewichtslage des Prüfmittels (1) im Frischbeton (9) gemessen wird, und dass aus der gemessenen Zeit ein Wert für die Fließeigenschaften, insbesondere die Viskosität, des Frischbetons (9) ermittelt wird.

6. Verfahren nach einem der Ansprüche 1 bis 5, **dadurch gekennzeichnet, dass** zur Temperaturkompensation eine Temperatur des Frischbetons (9) gemessen wird.

7. Verfahren nach einem der Ansprüche 1 bis 6, **dadurch gekennzeichnet, dass** der Wert für die Rohdichte des Frischbetons (9) direkt an einer Messmarke eines Schwimmkörpers (3) des Prüfmittels (1) abgelesen wird.

8. Verfahren nach Anspruch 7, **dadurch gekennzeichnet, dass** der Schwimmkörper (3) - in Gebrauchslage gesehen - derart mit einer nach unten gerichteten Spitze (31) ausgebildet ist, dass die Dauer bis zur Ausbildung der Gleichgewichtslage nur wenig durch die Spitze (31) beeinflusst wird.

9. Verfahren nach Anspruch 7 oder 8, **dadurch gekennzeichnet, dass** der, insbesondere kegel- oder pyramidenförmig ausgebildete, Schwimmkörper (3) mit - in Gebrauchslage gesehen - einem von unten nach oben stetig ansteigenden horizontalen Querschnitt verwendet wird.

## Claims

1. A method for determining the quality of wet concrete (9), especially of self-compacting wet concrete (9), with a testing means (1) with an immersion body (2) for immersion into the wet concrete (9) being held against a wet concrete surface (91) with the bottom side of the testing means, as seen in the position of use, with the testing means (1) being released for immersion into the wet concrete (9), with an immersion depth of the testing means (1) being measured substantially after the formation of an equilibrium position, and with a value for the gross density of the wet concrete (9) being determined from the immersion depth, **characterized in that** the method is performed at least one second time after a predeterminable period of time for testing the venting and/or sedimentation stability.

2. A method according to claim 1, **characterized in that** the method is performed at least one second time after five to ten minutes.

3. A method according to claim 2, **characterized in that** the method is performed at least one second time after seven to 8 minutes.

4. A method according to one of the claims 1 to 3, **characterized in that** the method is performed a second time at the same position in the wet concrete (9).

5. A method according to one of the claims 1 to 4, **characterized in that** the time is measured until the formation of the equilibrium position of the testing means (1) in the wet concrete (9), and a value for the flow properties, especially the viscosity, of the wet concrete (9) is obtained from the measured time.

6. A method according to one of the claims 1 to 5, **characterized in that** a temperature of the wet concrete (9) is measured for temperature compensation.

7. A method according to one of the claims 1 to 6, **characterized in that** the value for the gross density of the wet concrete (9) is read directly on a measuring mark of a floating body (3) of the testing means (1).

8. A method according to claim 7, **characterized in that** the floating body (3), when seen in the position of use, is arranged in such a way with a downwardly facing tip (31) that the duration until the formation of the equilibrium position is influenced only to a low extent by the tip (31).

9. A method according to claim 7 or 8, **characterized in that** the floating body (3), which is especially arranged in a conical or pyramidal way, is used with a cross section which increases continually from the bottom to the top, as seen in the position of use.

## Revendications

1. Procédé pour déterminer la qualité de béton frais (9), en particulier de béton frais autocompactant (9), dans lequel un moyen de contrôle (1) avec une sonde plongeante (2) destinée à être plongée dans le béton frais (9) est tenu avec le côté inférieur du moyen de contrôle, vu dans la position d'utilisation, sur une surface en béton frais (91), dans lequel le moyen de contrôle (1) est libéré pour pénétrer dans le béton frais (9), dans lequel une profondeur de pénétration du moyen de contrôle (1) est mesurée pour l'essentiel après qu'une situation d'équilibre est atteinte, et dans lequel la profondeur de pénétration sert à déterminer la masse volumique du béton frais (9), **caractérisé en ce qu'**afin de vérifier la stabilité de dégazage et/ou de sédimentation, le procédé est exécuté au moins une deuxième fois après un intervalle de temps pouvant être prédéterminé.

2. Procédé selon la revendication 1, **caractérisé en ce que** le procédé est exécuté au moins une deuxième fois après 5 à 10 minutes.

3. Procédé selon la revendication 2, **caractérisé en ce que** le procédé est exécuté au moins une deuxième fois après 7 à 8 minutes.

4. Procédé selon l'une des revendications 1 à 3, **caractérisé en ce que** le procédé est exécuté une deuxième fois au même endroit dans le béton frais (9).

5. Procédé selon l'une des revendications 1 à 4, **caractérisé en ce que** le temps jusqu'à la constitution de la situation d'équilibre du moyen de contrôle (1) dans le béton frais (9) est mesuré et **en ce que** le temps mesuré sert à déterminer une valeur pour les propriétés rhéologiques, en particulier la viscosité, du béton frais (9).

6. Procédé selon l'une des revendications 1 à 5, **caractérisé en ce qu'**une température du béton frais (9) est mesurée en vue de la compensation de la température.

7. Procédé selon l'une des revendications 1 à 6, **caractérisé en ce que** la valeur de masse volumique du béton frais (9) est lue directement sur une marque de mesure d'un flotteur (3) du moyen de contrôle (1).

8. Procédé selon la revendication 7, **caractérisé en ce que** le flotteur (3) est conformé avec une pointe (31) orientée vers le bas, vue dans la position d'utilisation, de telle façon que la pointe (31) n'influence que peu le délai avant l'établissement de la position d'équilibre.

9. Procédé selon la revendication 7 ou 8, **caractérisé en ce que** le flotteur (3) utilisé, de forme en particulier conique ou pyramidale, a une section horizontale augmentant de façon continue de bas en haut, vu dans la position d'utilisation.
